(19)

Europäisches
Patentamt
European
Patent Office
Office européen
des brevets

(11) **EP 1 860 070 A1**

(12) **EUROPEAN PATENT APPLICATION**

(43) Date of publication:
**28.11.2007 Bulletin 2007/48**

(51) Int Cl.:
*C01G 23/047* (2006.01)    *C09C 1/36* (2006.01)

(21) Application number: **07016268.0**

(22) Date of filing: **01.12.2000**

(84) Designated Contracting States:
**AT BE CH CY DE DK ES FI FR GB GR IE IT LI LU MC NL PT SE TR**

(30) Priority: **01.12.1999 GB 9928438**

(62) Document number(s) of the earlier application(s) in accordance with Art. 76 EPC:
**00977761.6 / 1 233 930**

(71) Applicant: **ISIS INNOVATION LIMITED Summertown, Oxford OX2 7SG (GB)**

(72) Inventors:
 • **Knowland, John Sebastian Oxford, OX2 7AX (GB)**

 • **Dobson, Peter James Oxford, OX1 3PJ (GB)**
 • **Walkefield, Gareth Yarnton Kidlington Oxford, OX5 1PF (GB)**

(74) Representative: **Benson, John Everett J. A. Kemp & Co. 14 South Square Gray's Inn London WC1R 5JJ (GB)**

Remarks:
This application was filed on 20 - 08 - 2007 as a divisional application to the application mentioned under INID code 62.

(54) **A particle comprising a host lattice and a guest, its preparation and use in ultraviolet light screening compositions**

(57)    A UV screening composition comprising novel particles which are capable of absorbing UV light so that electrons and positively charged holes are formed within the particles is disclosed. These particles comprise a host lattice incorporating a second component to provide luminescence trap sites and/or killer sites, said second component being niobium, vanadium, antimony, tantalum, strontium, calcium, magnesium, barium, molybdenum or silicon.

**EP 1 860 070 A1**

**Description**

The present invention relates to UV screening compositions, methods for their preparation and their use. The invention in particular relates to, for example, compositions comprising particulate oxides, their preparation and their use as, for example, paints, plastics, coatings, pigments, dyes and compositions for topical application, in particular, for example, sunscreens.

The effects associated with exposure to sunlight are well known. For example, painted surfaces may become discoloured and exposure of skin to UVA and UVB light may result in, for example, sunburn, premature ageing and skin cancer.

Commercial sunscreens generally contain components which are able to reflect and/or absorb UV light. These components include, for example, inorganic oxides such as zinc oxide and titanium dioxide.

Titanium dioxide in sunscreens is generally formulated as "micronised" or "ultrafine" (20-50 nm) particles (so-called microreflectors) because they scatter light according to Rayleigh's Law, whereby the intensity of scattered light is inversely proportional to the fourth power of the wavelength. Consequently, they scatter UVB light (with a wavelength of from 290 to 320 nm) and UVA light (with a wavelength of from 320 to 400 nm) more than the longer, visible wavelengths, preventing sunburn whilst remaining invisible on the skin.

However, titanium dioxide also absorbs UV light efficiently, catalysing the formation of superoxide and hydroxyl radicals which may initiate oxidations. The crystalline forms of $TiO_2$, anatase and rutile, are semiconductors with band gap energies of about 3.23 and 3.06 eV respectively, corresponding to light of about 385 nm and 400 nm (I eV corresponds to 8066 $cm^{-1}$).

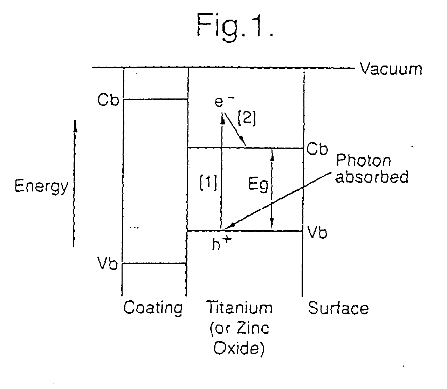

An incident photon is absorbed by titanium dioxide if its energy is greater than the semiconductor band gap Eg shown in Figure 1. As a result an electron from the valence band (Vb) is promoted into the conduction band (Cb) (transition [1]). If the energy of the incident photon is less than Eg it will not be absorbed as this would require that the electron be promoted to within the band gap and this energy state is forbidden. Once promoted, the electron relaxes to the bottom of the conduction band (transition [2]) with the excess energy being emitted as heat to the crystal lattice.

When the electron is promoted it leaves behind a hole which acts as a positive particle in the valence band. Both the electron and the hole are then free to migrate around the titanium dioxide particle. The electron and hole may recombine emitting a photon of energy equal to the band gap energy. However, the lifetime of the electron/hole pair is quite long due to the specific nature of the electronic band structure. Thus there is sufficient time (ca. $10^{-11}$s) for the electron and hole to migrate to the surface and react with absorbed species.

In aqueous environments, the electrons react with oxygen, and the holes with hydroxyl ions or water, forming superoxide and hydroxyl radicals:

$$TiO_2 + h\upsilon \rightarrow TiO_2(e^-/h^+) \rightarrow e^-(Cb) + h^+(Vb)$$

$$e^-(Cb) + O_2 \rightarrow O_2^{\cdot-} \rightarrow HO_2^{\cdot}$$

$$h^+(Vb) + OH^- \rightarrow {\cdot}OH$$

This has been studied extensively in connection with total oxidation of environmental pollutants, especially with anatase, the more active form [A. Sclafani et al., J.Phys. Chem., (1996), 100, 13655-13661].

It has been proposed that such photo-oxidations may explain the ability of illuminated titanium dioxide to attack biological molecules. Sunscreen titanium dioxide particles are often coated with compounds such as alumina, silica and zirconia which form hydrated oxides which can capture hydroxyl radicals and may therefore reduce surface reactions. However, some $TiO_2/Al_2O_3$ and $TiO_2/SiO_2$ preparations exhibit enhanced activity [C. Anderson et al., J. Phys. Chem., (1997), 101, 2611-2616].

As titanium dioxide may enter human cells, the ability of illuminated titanium dioxide to cause DNA damage has also recently been a matter of investigation. It has been shown that particulate titanium dioxide as extracted from sunscreens and pure zinc oxide will, when exposed to illumination by a solar simulator, give rise to DNA damage both *in vitro* and in human cells [R. Dunford et al, FEBS Lett., (1997), 418, 87-90]. In our application No. PCT/WO99/60994 we describe and claim particles which comprise a host lattice incorporating a second component to provide luminescence trap sites and/or killer sites. The host lattice is typically $TiO_2$ and the second component is preferably manganese, but other metals namely nickel, iron, chromium, copper, tin, aluminium, lead, silver, zirconium, zinc, cobalt and gallium are also mentioned.

According to the present invention other metals have been found to be effective. These are gallium, niobium, for example $Nb^{5+}$, vanadium, for example $V^{3+}$ or $V^{5+}$, antimony, for example $Sb^{3+}$, tantalum, for example $Ta^{5+}$, strontium, calcium, magnesium, barium, molybdenum, for example $Mo^{3+}$, $Mo^{5+}$ or $Mo^{6+}$ and silicon ions. These metals may be incorporated singly or in combination of 2 or 3 or more either with themselves or with any of the metals disclosed above including $Sn^{4+}$, $Mn^{2+}$, $Mn^{3+}$ and $Co^{2+}$. Accordingly the present invention provides a particle which comprises a host lattice incorporating a second component to provide luminescence trap sites and/or killer sites, said second component being niobium, vanadium, antimony, tantalum, strontium, calcium, magnesium, barium, molybdenum or silicon. The host lattice is preferably selected from oxides, especially, for example $TiO_2$ and ZnO, or, for example, phosphates, titanates, silicates, aluminates, oxysilicates, tungstates and molybdenates. Preferred particles according to the present invention comprise a titanium dioxide host lattice doped with vanadium ions in the 5+ state.

It is believed that the presence of the second component such as vanadium ions may make the host lattice more p-type. When a p-type particle absorbs light generating electrons and holes it is believed that any excess electrons are recombined within the particle and so are prevented from leaving the particle and inducing reactions which may result in DNA damage.

It is also believed that with such particles the production of hydroxyl radicals is substantially reduced. Thus the production of hydroxyl radicals may be substantially prevented. The minimisation of migration to the surface of the particles of the electrons and/or the positively charged holes may be tested by, for example, looking for a reduction in the number of strand breaks inflicted on DNA by light in the presence of particles according to the present invention, as compared with the number of strand breaks observed in DNA on treatment with particles used in conventional sunscreen compositions and light, or light alone.

The average primary particle size of the particles is generally from about 1 to 200 nm, for example about 50 to 150 nm, preferably from about 1 to 100 nm, more preferably from about 1 to 50 nm and most preferably from about 20 to 50 nm. For example, in sunscreens the particle size is preferably chosen to avoid colouration of the final product. For this purpose particles of about 50 nm or less may be preferred especially, for example, particles of about 3 to 20 nm, preferably about 3 to 10 nm, more preferably about 3 to 5 nm.

Where particles are substantially spherical then particle size will be taken to represent the diameter. However, the invention also encompasses particles which are non-spherical and in such cases the particle size refers to the largest dimension.

The optimum amount of the second component in the host lattice may be determined by routine experimentation. It will be appreciated that the amount of the second component may depend on the use of the particles. For example, when the particles are used in UV screening compositions for topical application, it may be desirable for the amount of the second component in the host lattice to be low so that the particles are not coloured. Amounts as low as 0.1% or less, for example 0.05%, or as high as 1% or above, for example 5% or 10%, can generally be used.

The dopant ions may be incorporated into the host lattice by a baking technique typically at a temperature of at least 300°C, generally at least 400°C and usually at least 600°C, for example 600°C to 1000°C, especially 650°C to 750°C eg. about 700°C. Thus, for example, these particles may be obtained in a known manner by combining particles of a host lattice with a second component in the form of a salt such as a chloride or an oxygen-containing anion such as a perchlorate or a nitrate, in solution or suspension, typically in solution in water, and then baking it. A sufficient time should be allowed for the incorporation to be complete. Typically at least one hour is required, for example about 3 hours. Increasing the time further has generally little further effect.

Other routes which may be used to prepare the doped materials include a precipitation process of the type described in J. Mat. Sci. (1997) 36, 6001-6008. In this process solutions of the dopant salt and of an alkoxide of the host metal are mixed. The mixed solution is then heated to convert the alkoxide to the oxide. Heating is continued until a precipitate of the doped material is obtained. It will be appreciated that the precise temperatures of heating will depend on the nature of the alkoxide. Further, in the case of a titanium alkoxide, for example titanium isopropoxide, temperature will dictate whether the resulting titanium dioxide is in the anatase or rutile form. Generally, a higher temperature is used to obtain the anatase form.

It is believed that the dopant ions (vanadium is taken as an example) within the absorbing core act as localised sites and as such may exist within the band gap. Transitions [1] and [2] may occur as shown in Figure 1. However, the electron and hole may then relax to the excess $V^{5+}$ sites. Thus the electrons and holes may be trapped so that they cannot migrate to the surface of the particles and react with absorbed species. The electrons and holes may then recombine at the $V^{5+}$ sites accompanied by the release of a photon with an energy equivalent to the difference in the energy levels.

When the host is titanium dioxide it has been found that the presence of the second component enhances the conversion from anatase to rutile on baking; it appears that, surprisingly, the dopant ion has the effect of catalysing the conversion. It is believed that the dopant ion must be present in the lattice to achieve this result. Thus on heating to at least, say, 530°C or 540°C, for example 600°C, at least 90% and generally at least 95%, for example 96 to 98%, of the anatase has been converted to the rutile form. The rutile form of titania is known to be more photostable than the anatase form. Heating undoped anatase to the same temperature results in significantly less conversion to rutile. Anatase starts to be converted to the rutile form at about 530°C to 540°C; although the extent of conversion increases as the temperature is raised, it remains only partial.

The particles of the present invention may have an inorganic or organic coating. For example, the particles may be coated with oxides of elements such as aluminium, zirconium or silicon. The particles of metal oxide may also be coated with one or more organic materials such as polyols, amines, alkanolamines, polymeric organic silicon compounds, for example, $RSi[\{OSi(Me)_2\}xOR^1]_3$ where R is $C_1$-$C_{10}$ alkyl, $R^1$ is methyl or ethyl and x is an integer of from 4 to 12, hydrophilic polymers such as polyacrylamide, polyacrylic acid, carboxymethyl cellulose and xanthan gum or surfactants such as, for example, TOPO.

The present invention also provides a UV screening composition which comprises particles of the present invention and a carrier. In another aspect the present invention provides a method for preparing the compositions of the present invention which comprises associating the particles described above with a carrier. The compositions of the invention may be used in a wide range of applications where UV screening is desired including paints, plastics, coatings and dyes, but are particularly preferred for topical application. The compositions for topical application may be, for example, cosmetic compositions including lipsticks, skin anti-ageing compositions in the form of, for example, creams, skin lightening compositions in the form of, for example, face powders and creams, compositions for protecting the hair and, preferably, sunscreens. Compositions of the present invention may be employed as any conventional formulation providing protection from UV light.

In effect the compositions of the present invention may be used to screen or protect a substrate from UV light as, for example, in sunscreens and/or screen or protect a UV sensitive component in the composition such as octyl methoxycinnamate, butyl methoxydibenzoyl methane or any of the following compounds:

(a) Para-aminobenzoic acids, esters and derivatives thereof, for example, 2-ethylhexyl para-dimethylaminobenzoate;
(b) methoxycinnamate esters such as 2-ethylhexyl para-methoxycinnamate, 2-ethoxyethyl para-methoxycinnamate or $\alpha,\beta$-di-(para-methoxycinnamoyl)-$\alpha'$-(2-ethylhexanoyl)-glycerin;
(c) benzophenones such as oxybenzone;
(d) dibenzoylmethanes such as 4-tert-butyl-4'methoxydibenzoylmethane;
(e) 2-phenylbenzimidazole-5 sulfonic acid and its salts;
(f) alkyl-$\beta,\beta$-diphenylacrylates for example askyl $\alpha$-cyano-$\beta$, $\beta$-diphenylacrylates such as octocrylene;
(g) triazines such as 2,4,6-trianilino-(p-carbo-2-ethyl-hexyl-1-oxy)-1,3,5 triazine;
(h) camphor derivatives such as methylbenzylidene camphor.
(i) organic pigments sunscreening agents such as methylene bis-benzotriazole tetramethyl butylphenol;
(j) silicone based sunscreening agents such-as dimethicodiethyl benzal malonate.

In compositions for topical application, the metal oxides are preferably present at a concentration of about 0.5 to 10 % by weight, preferably about 3 to 8 % by weight and more preferably about 5 to 7 % by weight. Such compositions may comprise one or more of the compositions of the present invention.

The compositions for topical application may be in the form of lotions, e.g. thickened lotions, gels, vesicular dispersions, creams, milks, powders, solid sticks, and may be optionally packaged as aerosols and provided in the form of foams or sprays.

The compositions may contain, for example, fatty substances, organic solvents, silicones, thickeners, demulcents, other UVA, UVB or broad-band sunscreen agents, antifoaming agents, moisturizing agents, perfumes, preservatives, surface-active agents, fillers, sequesterants, anionic, cationic, nonionic or amphoteric polymers or mixtures thereof, propellants, alkalizing or acidifying agents, colorants and metal oxide pigments with a particle size of from 100 nm to 20000 nm such as iron oxides.

The organic solvents may be selected from lower alcohols and polyols such as ethanol, isopropanol, propylene glycol, glycerin and sorbitol.

The fatty substances may consist of an oil or wax or mixture thereof, fatty acids, fatty acid esters, fatty alcohols, vaseline, paraffin, lanolin, hydrogenated lanolin or acetylated lanolin.

The oils may be selected from animal, vegetable, mineral or synthetic oils and especially hydrogenated palm oil, hydrogenated castor oil, vaseline oil, paraffin oil, Purcellin oil, silicone oil and isoparaffin.

The waxes may be selected from animal, fossil, vegetable, mineral or synthetic waxes. Such waxes include beeswax, Camauba, Candelilla, sugar cane or Japan waxes, ozokerites, Montan wax, microcrystalline waxes, paraffins or silicone waxes and resins.

The fatty acid esters are, for example, isopropyl myristate, isopropyl adipate, isopropyl palmitate, octyl palmitate, $C_{12}$-$C_{15}$ fatty alcohol benzoates ("FINSOLV TN" from FINETEX), oxypropylenated myristic alcohol containing 3 moles of propylene oxide ("WITCONOL APM" from WITCO), capric and caprylic acid triglycerides ("MIGLYOL 812" from HULS).

The compositions may also contain thickeners which may be selected from cross-linked or non cross-linked acrylic acid polymers, and particularly polyacrylic acids which are cross-linked using a polyfunctional agent, such as the products sold under the name "CARBOPOL" by the company GOODRICH, cellulose, derivatives such as methylcellulose, hydroxymethylcellulose, hydroxypropyl methylcellulose, sodium salts of carboxymethyl cellulose, or mixtures of cetylstearyl alcohol and oxyethylenated cetylstearyl alcohol containing 33 moles of ethylene oxide.

When the compositions of the present invention are sunscreens they may be in a form of suspensions or dispersions in solvents or fatty substances or as emulsions such as creams or milks, in the form of ointments, gels, solid sticks or aerosol foams. The emulsions may further contain anionic, nonionic, cationic or amphoteric surface-active agents. They may also be provided in the form of vesicular dispersions of ionic or nonionic amphiphilic lipids prepared according to known processes.

Particularly when the particles are of titanium dioxide they are useful as pigments in paints. It is known that paints and varnishes undergo significant degradation in the presence of sunlight and/or UV light. The antioxidants currently used to counteract this are not wholly effective. The use of the inactivated titanium dioxide particles of the present invention significantly reduces the degradation of paints and varnishes and, in addition, contributes to a reduction in the "yellowing" of white formulations which occurs even in the dark but which is believed to be free radical initiated. Paint formulations generally comprise pigments, binders or resins, solvents, and additives. The choice of binder or resin has a significant effect upon the performance properties of the paint. The preferred properties of the binder include the ability to cure under various conditions, good adhesion to various substrates, abrasive resistance, flexibility and water resistance. Typical binders include latex emulsions, alkyds, linseed oil, oil-modified epoxy and polyurethane resins and water-reducible alkyd and oil-systems. Generally the pigment volume concentration i.e. the total volume of pigment divided by the total volume of pigment and binder, expressed as a percentage, is from 15 to 75. The solvent is usually selected for its compatibility with the binder, and because it has the desired evaporation rate and toxicity profile. Typical solvents include mineral spirits, glycol solvents and other organic solvents. Additives are usually also included to either fulfil functions that are not covered by the other components or to assist the binder and the pigments to fulfil their particular functions. Typical additives include thickeners, driers, pigment dispersants, surfactants, defoamers and biocides.

Brief Description of the Figures

Figure 1 shows the absorption of a photon of UV light by titanium dioxide as found in conventional sunscreens.

Figure 2 shows the effect on DNA of rutile $TiO_2$, undoped or doped with varying amounts of $V^{5+}$ obtained by the precipitation process. The results were obtained by illumination of DNA in vitro as described in WO 99/60994.

Figure 3 shows the effect on DNA of $V^{5+}$ doped $TiO_2$ obtained by the baking process.

Figure 4 shows the effect on DNA of $TiO_2$ doped with different dopants.

The Examples which follow further illustrate the present invention with reference to the figures.

Example 1

Preparation of vanadium doped titanium dioxide by baking

Titanium dioxide (25g) and ammonium vanadate (0.8g) were mixed in deionized water (100 ml). The resulting mixture was ultrasonicated for 10 minutes and then boiled dry. The material produced was fired at 700°C for 3 hours to give 1% vanadium doped titanium dioxide. Titanium dioxide particles with differing dopant levels were prepared in an analogous manner by varying the amount of ammonium vanadate.

Example 2

Preparation of doped rutile titanium dioxide by precipitation.

Water (720 ml; 40 moles) was mixed with concentrated hydrochloric acid (43 ml; 0.5 moles) and kept below 25°C. Isopropanol (50 ml; 0.65 moles) was added slowly keeping the temperature below 25 °C.

For 1% doping, 0.0005 moles of dopant was added to the mixture and stirred until fully dissolved. Thus for ammonium metavanadate (MW = 116.98) 0.05849g was added.

Titanium isopropoxide (15 ml; 0.05 moles) was added dropwise with vigorous stirring with a magnetic stirrer until a translucent solution was produced. The solution was placed in a water bath at room temperature and slowly heated to 50°C and held at that temperature. After 1 to 4 hours the solution will begin to go cloudy as precipitation begins.

The temperature was held at 50°C until the precipitate had all settled and the solution had cleared (approximately 3 - 4 hours). The material was removed from the heat and allowed to settle for 12 hours.

As much supernatant as possible was pipetted off and the precipitate harvested by repeated centrifugation. The precipitate was washed twice with a mixture of water (44.3 ml), concentrated hydrochloric acid (2.6 ml) and isopropanol (3.1 ml). 15 ml of fluid was retained from the second wash and the wet precipitate was re-suspended and freeze dried.

Example 3

Preparation of doped anatase titanium dioxide by precipitation.

The procedure of Example 2 was repeated except for the heating step. In order to obtain the anatase form, the solution was heated rapidly (5°C/min) up to 90 ° C and held there until precipitation was complete (approximately 3½ hours).

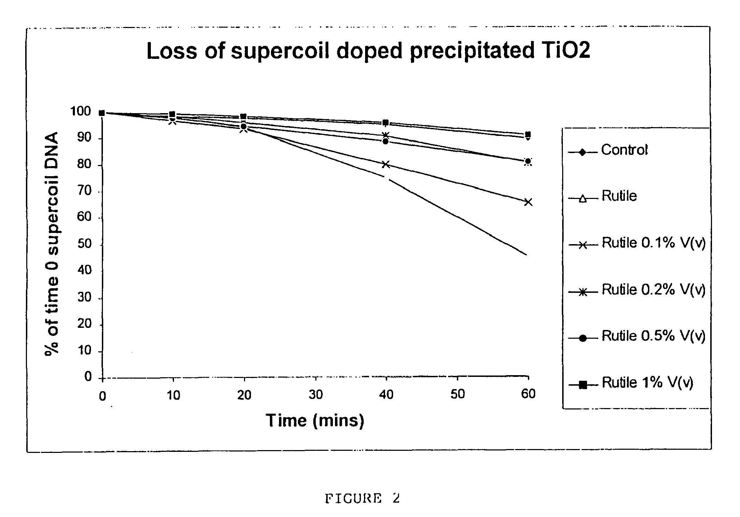

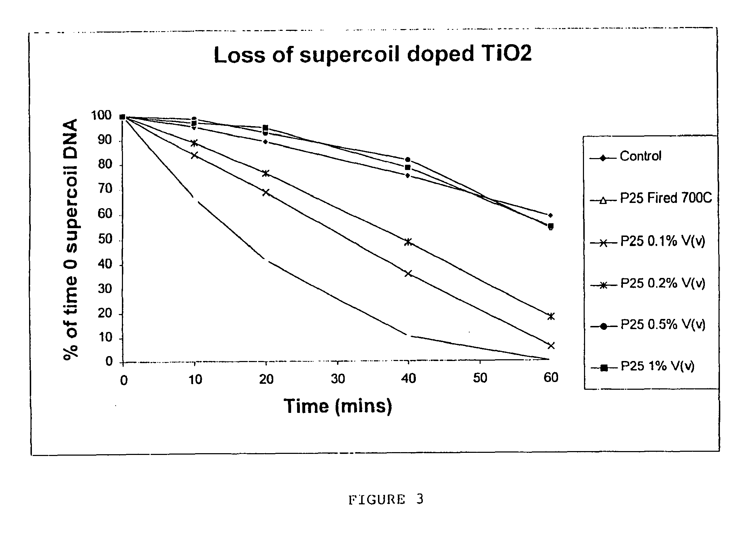

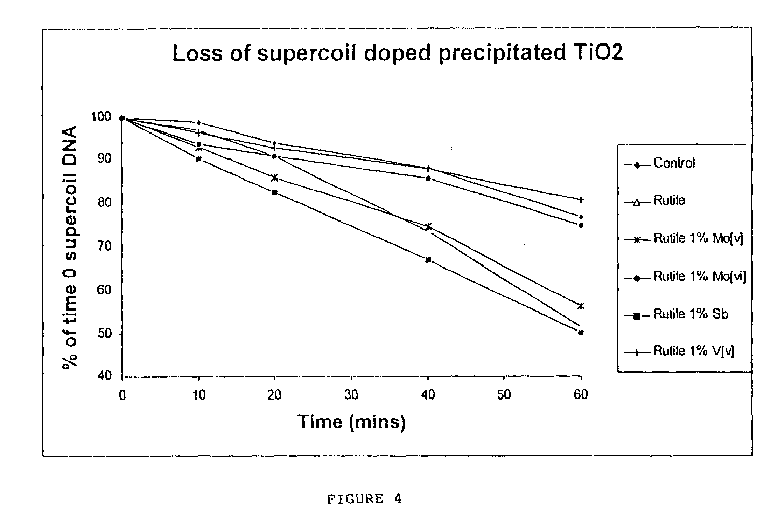

Figures 2, 3 and 4 show the effects obtained on DNA using these doped materials compared with undoped materials. P25 is a commercial grade of TiO$_2$.

Example 4

Preparation of oil-in-water sunscreen composition.

The following ingredients were used:

| Phase A | %ww |
| --- | --- |
| Glyceryl Stearate and PEG 100 Stearate | 5 |
| Sorbitan Stearate | 0.5 |
| Polysorbate 60 | 0.9 |
| Cetyl Alcohol | 1 |
| Liquid Paraffin | 8 |
| Sunflower oil | 5 |
| Dimethicone | 2 |
| Phase B | |
| Titanium Dioxide | 5 |
| Zinc Oxide | 2 |
| Xanthan Gum | 0.1 |
| Water | to 100 |

Phase A was heated to 70°C. To prepare Phase B, the Xanthan gum was dispersed into water and the resulting dispersion heated to 70°C. Using a high energy homogeniser the Titanium and Zinc were then dispersed into the hot Xanthan solution. Phase A was added to Phase B slowly and homogenised.

Example 5

Preparation of combined inorganic/organic sunscreen composition

The following ingredients were used:

| Phase A | %ww |
| --- | --- |
| Glyceryl Stearate and PEG 100 Stearate | 5 |
| Sorbitan Stearate | 0.5 |
| Polysorbate 60 | 0.9 |
| Cetyl Alcohol | 1 |
| Liquid Paraffin | 8 |
| Sunflower oil | 5 |
| Dimethicone | 2 |
| C12-15 Alcohols Benzoate | 5 |
| Octyl Methoxycinnamate | 4 |
| Butyl Methoxydibenzoyl Methane | 2 |
| Phase B | |
| Titanium Dioxide | 5 |
| Xanthan gum | 0.1 |
| Water | to 100 |

Phase A was heated to 70°C. To prepare Phase B, the xanthan gum was dispersed into water and the resulting dispersion heated to 70°C. Using a high energy homogeniser the Titanium Dioxide was then dispersed into the hot Xanthan solution.

Phase A was added to Phase B slowly and homogenised.

The following sunscreens are examples of organic filters that can be used in the above formulation to replace the methoxycinnamate and/or the dibenzoyl methane.

(a) Para-aminobenzoic acids, esters and derivatives thereof, for example, 2-ethylhexyl para-dimethylaminobenzoate;
(b) methoxycinnamate esters such as 2-ethylhexyl para-methoxycinnamate, 2-ethoxyethyl para-methoxycinnamate or $\alpha,\beta$-di-(para-methoxycinnamoyl)-$\alpha$'-(2-ethylhexanoyl)-glycerin;
(c) benzophenones such as oxybenzone;
(d) dibenzoylmethanes such as 4-tert-butyl-4'methoxydibenzoylmethane;
(e) 2-phenylbenzimidazole-5 sulfonic acid and its salts;
(f) alkyl-$\beta,\beta$-diphenylacrylates for example askyl $\alpha$-cyano-$\beta$, $\beta$-diphenylacrylates such as octocrylene;
(g) triazines such as 2,4,6-trianilino-(p-carbo-2-ethyl-hexyl-1-oxy)-1,3,5 triazine;
(h) camphor derivatives such as methylbenzylidene camphor.
(i) organic pigments sunscreening agents such as methylene bis-benzotriazole tetramethyl butylphenol;
(j) silicone based sunscreening agents such as dimethicodiethyl benzal malonate.

Example 6

Preparation of water-in-oil sunscreen composition

| Phase A | %ww |
| --- | --- |
| Dimethicone and trimethylsiloxysilicate | 5 |
| Cyclomethicone | 8 |
| Laurylmethicone copolyol | 3 |
| Liquid Paraffin | 5 |
| | |
| Phase B | |
| Glycerine | 5 |
| Sodium Chloride | 1 |
| Titanium dioxide | 3 |
| Zinc Oxide | 8 |
| Water | to 100 |

The titanium dioxide and zinc oxide were dispersed into the other components of Phase B using a high energy homogeniser. Phases A and B were each heated to 70°C, then Phase A was slowly added to Phase B with stirring. The resulting mixture was homogenised to give the required viscosity.

## Claims

1. A particle which comprises a host lattice incorporating a second component to provide luminescence trap sites and/or killer sites, said second component being niobium, vanadium, antimony, tantalum, strontium, calcium, magnesium, barium, molybdenum or silicon.

2. A particle according to claim 1 wherein the second component is vanadium in the 5+ state.

3. A particle according to claim 1 or 2 wherein the host lattice is a metal oxide selected from zinc oxide and titanium dioxide.

4. A particle according to any one of claims I to 3 wherein the particles comprise a titanium dioxide host lattice doped with vanadium in the 5+ state.

5. A particle according to any one of the preceding claims wherein the second component is present in an amount of from about 0.1 to 1%.

6. A particle according to any one of the preceding claims which has a size is from 1 to 200 nm.

7. A particle according to any one of the preceding claims which has an outer coating.

8. A particle according to claim 7 wherein the outer coating is of an oxide of aluminium, zirconium or silicon.

9. A particle according to claim 1 substantially as hereinbefore described.

10. A process for preparing a particle as claimed in any one of the preceding claims which comprises applying the second component as a salt to particles of the host and baking it.

11. A process according to claim 10 wherein baking is carried out at a temperature of at least 600°C.

12. A process according to claim 11 wherein baking is carried out at a temperature of 600°C to 1000°C.

13. A particle as defined in any one of claims 1 to 9 whenever prepared by a process as claimed in any one of claims 10 to 12.

14. A UV screening composition which comprises particles as claimed in any one of claims 1 to 9 and 13 and a carrier.

15. A composition according to claim 14 which is for topical application.

16. A composition according to claim 15 which is formulated for cosmetics use.

17. A composition according to claim 16 which is a sunscreen.

18. A composition according to claim 14 which is a paint.

19. Use of particles as claimed in any one of claims 1 to 9 and 13, in a sunscreen composition for topical application, to screen UV radiation whilst minimising DNA damage to the host.

20. Use of particles as claimed in any one of claims 1 to 9 and 13 as a UV screening agent which gives rise to reduced DNA damage.

# Fig.1.

FIGURE 2

EP 1 860 070 A1

## Loss of supercoil doped TiO2

Legend:
- —◆— Control
- —△— P25 Fired 700C
- —✗— P25 0.1% V(v)
- —✳— P25 0.2% V(v)
- —●— P25 0.5% V(v)
- —■— P25 1% V(v)

Y-axis: % of time 0 supercoil DNA

X-axis: Time (mins)

FIGURE 3

Loss of supercoil doped precipitated TiO2

FIGURE 4

EP 1 860 070 A1

**European Patent Office**

## EUROPEAN SEARCH REPORT

Application Number

EP 07 01 6268

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,P, X | WO 99/60994 A (KNOWLAND JOHN SEBASTIAN ;ISIS INNOVATION (GB); DOBSON PETER JAMES) 2 December 1999 (1999-12-02) * the whole document * ----- | 1-20 | INV. C01G23/047 C09C1/36 |
| X | US 5 451 252 A (ELFENTHAL LOTHAR ET AL) 19 September 1995 (1995-09-19) * column 2, line 45 - column 4, line 11 * * column 1, line 62 - column 2, line 2; claims 3,9-16 * | 1-9 | |
| A | * claims 9,10 * ----- | 10,13 | |
| P,X | PATENT ABSTRACTS OF JAPAN vol. 2000, no. 01, 31 January 2000 (2000-01-31) & JP 11 279019 A (SHISEIDO CO LTD), 12 October 1999 (1999-10-12) * abstract * ----- | 1-20 | |
| X | DE 25 45 243 A (METALLGESELLSCHAFT AG) 21 April 1977 (1977-04-21) * page 4, line 1 - line 24; claims 1-7 * * page 7, line 10 - line 21 * * page 7, line 24 - line 36 * ----- | 1-13 | TECHNICAL FIELDS SEARCHED (IPC) C01G C09C A61K C09K |
| A | US 3 981 737 A (EVILAMPI TOIVO ET AL) 21 September 1976 (1976-09-21) * abstract * ----- | 10-12 | |

The present search report has been drawn up for all claims

| Place of search | Date of completion of the search | Examiner |
|---|---|---|
| Munich | 10 October 2007 | Fortunati, Taddiano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 01 6268

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2007

| Patent document cited in search report | | Publication date | Patent family member(s) | | Publication date |
|---|---|---|---|---|---|
| WO 9960994 | A | 02-12-1999 | AU | 765456 B2 | 18-09-2003 |
| | | | AU | 4273299 A | 13-12-1999 |
| | | | DE | 69929799 T2 | 31-08-2006 |
| | | | EP | 1079796 A1 | 07-03-2001 |
| | | | ES | 2255292 T3 | 16-06-2006 |
| | | | JP | 2002516347 T | 04-06-2002 |
| | | | US | 6869596 B1 | 22-03-2005 |
| US 5451252 | A | 19-09-1995 | NONE | | |
| JP 11279019 | A | 12-10-1999 | NONE | | |
| DE 2545243 | A | 21-04-1977 | NONE | | |
| US 3981737 | A | 21-09-1976 | DE | 2407429 A1 | 22-08-1974 |
| | | | FI | 52351 B | 02-05-1977 |
| | | | FR | 2218367 A1 | 13-09-1974 |
| | | | GB | 1427453 A | 10-03-1976 |

EPO FORM P0459

For more details about this annex : see Official Journal of the European Patent Office, No. 12/82

**EP 1 860 070 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9960994 A

**Non-patent literature cited in the description**

- **A. SCLAFANI et al.** *J.Phys. Chem,* 1996, vol. 100, 13655-13661
- **C. ANDERSON et al.** *J. Phys. Chem,* 1997, vol. 101, 2611-2616
- **R. DUNFORD et al.** *FEBS Lett,* 1997, vol. 418, 87-90
- *J. Mat. Sci.,* 1997, vol. 36, 6001-6008